# FASCICULE DE BREVET EUROPEEN

(11) **EP 0 408 424 B1**
(45) Date de publication et mention de la délivrance du brevet: **04.05.1994**
(21) Numéro de dépôt: 90401938.7
(22) Date de dépôt: 04.07.1990
(51) Int. Cl.: G07D 7/00, A61B 5/117, G09F 3/02, C12Q 1/68, D21H 21/46

(54) **Procédé et dispositif de marquage crypté de haute securité pour la protection d'objets de valeur**
Vorrichtung und Verfahren zum verschlüsselten Sicherheitsmarkierung zum Schutz von Wertgegenständen
Method and device for high-security encyphered marking to protect high-value objects

(30) Priorité: 07.07.1989 FR 8909202
(43) Date de publication de la demande: 16.01.1991
(73) Titulaire: BIOPROBE SYSTEMS, F-75012 Paris (FR)
(72) Inventeur: Lebacq, Philippe, F-75012 Paris (FR)
(74) Mandataire: Netter, André

(56) Documents cités:
- EP-A- 0 090 130
- DE-A- 3 742 706

## Description

L'invention concerne le marquage d'objets de valeur, notamment d'oeuvres d'art ou de documents, en vue de leur protection contre le vol, la revente ou la contrefaçon.

Elle concerne plus particulièrement un procédé et un dispositif de marquage crypté, propres à permettre le marquage d'objets de valeur et à permettre, ultérieurement, et en cas de besoin, d'identifier ou d'authentifier lesdits objets, et cela de manière irréfutable.

Par l'expression "marquage crypté", on entend désigner présentement un marquage dissimulé et invisible dans un endroit caché de l'objet, connu seulement de son propriétaire et indétectable par les tiers.

L'invention s'applique tout particulièrement à l'identification d'oeuvres d'art, telles que : peintures, sculptures, céramiques, poteries, vases, meubles, vêtements anciens, tapis, tapisseries, livres anciens, gravures, lithographies, objets de collection, etc.

Elle s'applique également à l'identification des biens d'équipement, tels que véhicules automobile, chaînes haute-fidélité, ordinateurs, téléviseurs, postes de radio, magnétoscopes, caméras, appareils photographiques, etc.

L'invention s'applique aussi à la protection contre la contrefaçon de toutes sortes de documents et de papiers officiels, notamment de documents de banque, chèques, contrats, testaments, papier monnaie, papiers d'identité, papiers juridiques, etc.

Il existe actuellement très peu de moyens pour protéger des objets de valeur, tels que oeuvres d'art ou autres biens mobiliers, contre le vol, la revente ou la contrefaçon. De plus, pour un propriétaire, il est très difficile de pouvoir apporter la preuve irréfutable que le bien volé, revendu ou contrefait, lorsqu'il est retrouvé, lui appartenait.

Une autre difficulté provient du fait que toutes les oeuvres d'art ne sont pas inventoriées. S'il en est ainsi, des toiles de maîtres par exemple, il n'en est pas de même pour les meubles notamment.

En France, sur le plan juridique, l'article 2279 du Code Civil dispose :
"En fait de meubles, la possession vaut titre. Néanmoins, celui qui a perdu ou auquel il a été volé une chose ou un objet, peut le revendiquer pendant trois ans à compter du jour de la perte ou du vol, contre celui dans les mains duquel il la trouve ; sauf à celui-ci son recours contre celui duquel il la tient".

Dans ces conditions, il est absolument nécessaire, en cas de perte ou de vol, de pouvoir rapporter la preuve de la propriété de son bien lorsque celui-ci est retrouvé entre les mains d'un tiers.

Parmi les moyens existant actuellement pour protéger les objets de valeur, notamment les oeuvres d'art, on connaît essentiellement l'utilisation d'implants métalliques que le propriétaire de l'objet insère dans un endroit particulier de l'objet, connu seulement de lui-même.

Ainsi, en cas de perte ou de vol, si l'objet est retrouvé entre les mains d'un tiers, le propriétaire peut théoriquement faire la preuve de cette propriété en allant retirer l'implant ou en faisant effectuer une radiographie de l'objet dans la région qui contient cet implant.

Malheureusement, un tel procédé n'est pas absolument fiable puisque l'implant peut être repéré par les tiers et cela par l'utilisation de rayons X ou de rayons gamma.

On a proposé également d'appliquer, sur un objet à protéger, un composé chimique choisi, ou composé-cible, propre à être détecté ultérieurement par tout moyen approprié, en particulier par un autre composé-chimique, ou composé-sonde.

Ainsi la Demande de Brevet européen No 0 090 130 propose un procédé d'authentification d'un document au moyen d'un système de réaction colorée utilisant une substance formatrice de couleur et une substance révélatrice. L'une des deux substances est appliquée sur l'objet, tandis que l'autre substance est utilisée à des fins de détection. Lorsque les deux substances sont mises en contact, il se produit une réaction colorée caractéristique qui permet d'authentifier le document.

Un tel procédé est, lui aussi, peu fiable étant donné le nombre limité de systèmes de réaction colorée susceptibles d'être utilisés.

C'est, en conséquence, un but de l'invention de procurer un procédé capable de faire la preuve irréfutable de la propriété d'un objet de valeur, notamment d'une oeuvre d'art.

C'est également un but de l'invention de procurer un procédé de marquage crypté capable de garantir l'identification de l'objet de valeur, le marquage par lui-même pouvant être délivré et réalisé par un expert et cela, à l'aide du procédé de l'invention.

L'invention a également pour but de procurer un procédé propre à identifier des papiers ou documents officiels, tels que contrats, actes, testaments, papiers officiels, papier monnaie, chèques, actions, etc.

Un autre but de l'invention est de procurer un tel procédé de marquage crypté dans lequel le marquage de l'objet de valeur ne peut être détecté par les tiers, notamment par des procédés de détection aux rayons X ou aux rayons gamma.

Enfin, un autre but de l'invention est de procurer un dispositif de marquage crypté propre à la mise en oeuvre du procédé de l'invention.

L'invention propose de réaliser le marquage crypté d'objets de valeur, et cela en utilisant certains des progrès spectaculaires que la biologie a fait depuis quelques années et qui rendent désormais disponibles des outils moléculaires d'une très grande efficacité. Parmi ceux-ci, la technologie des sondes nucléiques représente certainement l'outil le plus séduisant et le plus puissant.

La technologie des sondes nucléiques repose sur l'importante affinité qui existe entre deux brins d'acide nucléique complémentaires. Cette affinité est parfaitement illustrée par les estimations théoriques de la constante de dissociation (Kd) d'un hybride, encore appelé "duplex", de 273 paires de bases.

Cette valeur est de l'ordre de 10⁻²³ M à 5°C au-dessous de la température de demi-dénaturation du duplex (Tm). A titre de comparaison, les réactions antigènes-anticorps ont un Kd compris entre 10⁻⁵ et 10⁻⁹ M.

Selon cette technologie, il est possible de construire des sondes nucléiques (acides nucléiques-sondes) de séquences complémentaires de celles d'acides nucléiques-cibles et de réaliser la formation de duplex in vitro.

Si la sonde est marquée, soit radioactivement, soit non radioactivement, il est alors possible de détecter le duplex ainsi formé.

L'utilisation de sondes nucléiques permet ainsi de détecter les organismes vivants (ces derniers sont normalement tous porteurs d'acides nucléiques représentant leur patrimoine génétique), si l'on connaît la séquence ou une partie de la séquence des acides nucléiques desdits organismes.

La sonde marquée au préalable et propre à s'hybrider à l'acide nucléique-cible renfermé par l'organisme considéré, conduit à l'obtention d'un duplex ou hybride, dont le marquage permet la détection.

Il est ainsi possible de détecter de nombreux virus, bactéries, parasites, champignons, etc dans les milieux biologiques.

Les sondes nucléiques permettent également l'étude génétique d'organismes vivants plus complexes, comme les végétaux, les animaux et l'être humain.

Ces études permettent par exemple l'étude et la prévention des maladies génétiques, l'identification du père ou de la mère dans les recherches de paternité, l'identification d'un violeur ou d'un criminel parmi des suspects, etc.

Ainsi, jusqu'à présent, les sondes nucléiques ont été utilisées uniquement pour des applications médicales, agro-alimentaires ou scientifiques, pour lesquelles les acides nucléiques et notamment les sondes nucléiques représentent un outil de choix.

La Demanderesse a constaté, de manière surprenante, que la technologie des sondes nucléiques pouvait être utilisée avec succès dans la protection des objets de valeur, notamment des oeuvres d'art, pour apporter, et cela d'une manière absolument irréfutable, la preuve de la propriété d'objets volés revendus ou contrefaits, à condition que ceux-ci aient été au préalable marqués par le procédé de l'invention.

L'invention concerne, plus particulièrement, un procédé de marquage crypté de haute sécurité pour la protection d'objets de valeur dans lequel on applique sur un objet à marquer un composé chimique-cible propre à être détecté ultérieurement par un moyen de détection approprié.

Selon l'invention, ce procédé est caractérisé en ce qu'il comprend les opérations consistant à :
a) préparer une solution d'un acide nucléique-cible de séquence choisie formant motif de marquage et possédant un degré de fluidité choisi,
b) incorporer une quantité choisie de ladite solution à chaque objet d'une classe d'objets à marquer,
   ce qui permet de procéder ultérieurement, en cas de besoin, à une opération
c) d'identification ou authentification de l'objet en utilisant un moyen de détection dudit acide nucléique pour révéler le marquage.

Ainsi, l'invention consiste en l'utilisation de fragments d'acides nucléiques (appelés acides nucléiques-cibles) particuliers par leur séquence, leur taille et leur nature et capables d'être utilisés comme-cible de détection dans des objets de valeur, tels que des oeuvres d'art, des biens d'équipement, des papiers officiels ou contractuels, etc. Un acide nucléique-cible peut, en effet, être aisément caché pour être ultérieurement détecté directement ou par hybridation et apporter la preuve, par cette détection, de la propriété ou de l'authenticité d'un objet de valeur.

Il faut remarquer aussi que l'utilisation d'acides nucléiques est particulièrement avantageuse car ces derniers ont une solidité et une longévité exceptionnelles.

On peut faire observer, en effet, qu'un acide nucléique, notamment l'ADN, est une molécule particulièrement résistante, notamment en milieu sec. Un exemple illustre parfaitement cette solidité et même la longévité de l'ADN. Il s'agit de l'ADN prélevé sur des momies égyptiennes, qui a été cloné par génie génétique, amplifié puis enfin séquencé pour permettre l'étude de certains des gènes de momies vieilles de plus de 5000 ans.

Conformément à l'invention, l'acide nucléique-cible peut être facilement incorporé à l'objet à protéger. Ainsi, il peut être caché dans une oeuvre d'art, un meuble, une voiture, un bien quel qu'il soit, ou incorporé dans un papier, un contrat, une pièce officielle.

Dans une forme de réalisation préférée de l'invention, l'opération b) consiste à appliquer la solution d'acide nucléique sur une surface d'un support pour l'imprégner de ladite solution et incorporer ensuite le support ainsi imprégné dans chaque objet à marquer.

Comme matière de support, on peut utiliser, par exemple, de la nitrocellulose, du polyamide (nylon) chargé positivement ou non chargé, du papier, du carton, du bois, de la matière plastique, du tissu, du métal, du verre, de la céramique, de la terre cuite, une matière organique sous forme de billes ou de gel, ou encore une matière minérale.

On préfère tout particulièrement utiliser comme support une membrane de petite dimension susceptible d'être insérée soit à plat dans un objet mince, soit encore à l'état enroulé dans un objet plus épais.

Avantageusement, l'acide nucléique-cible est appliqué sur un support de nitrocellulose ou de nylon, chargé positivement ou non, et présentant de faibles dimensions. Par exemple, il peut s'agir d'une membrane de 5 mm de largeur pour 15 mm de longueur. Ce support, une fois imprégné de l'acide nucléique-cible, peut être roulé ou plié, collé ou incorporé dans la pièce à marquer.

En variante, l'opération b) peut consister à appliquer la solution d'acide nucléique-cible directement sur une surface de l'objet à marquer et cela, dans le cas où le matériau constitutif de ladite surface permet un accrochage direct dudit acide nucléique.

Dans l'opération b) on applique avantageusement la solution d'acide nucléique sous la forme d'un marquage en écrivant un numéro, une lettre, un mot, une phrase, une signature ou tout autre signe de marquage.

Dans une forme préférée de l'invention, dans l'opération c), on utilise, comme moyen de détection, une sonde d'acide nucléique simple brin de séquence complémentaire de celle de l'acide nucléique-cible incorporé à l'objet et capable de s'hybrider à celle-ci, ce qui permet une détection spécifique de l'acide nucléique-cible par hybridation moléculaire.

L'acide nucléique-cible et l'acide nucléique-sonde peuvent avoir différentes longueurs, différentes séquences et être de différentes natures. Il peut s'agir d'ADN ou d'ARN d'origine naturelle ou synthétique, ou encore de dérivés de synthèse contenant en tout ou en partie des bases rares ou des bases modifiées.

On peut aussi utiliser, comme séquence d'acide nucléique-cible, une séquence du génome ou le génome entier d'une personne humaine, par exemple du propriétaire de l'objet à marquer. De cette manière on pourra ensuite, par hybridation nucléique, identifier le propriétaire comme cela est réalisé dans les tests de paternité (DNA Fingerprint) grâce à des sondes dites satellites ou répétées ou tout autre type de sonde susceptible de générer un polymorphisme identifiant le propriétaire d'une manière irrévocable.

De manière plus générale, on peut utiliser dans l'invention tout acide nucléique-cible susceptible de générer un polymorphisme tel qu'il puisse être détecté par des sondes satellites, des sondes répétées, etc, et toute sonde capable de détecter un polymorphisme.

Dans une forme de réalisation préférée de l'invention, l'acide nucléique-cible et l'acide nucléique-sonde sont des oligonucléotides complémentaires dont les séquences sont obtenues de façon aléatoire, par exemple au moyen d'un programme informatique.

Ainsi, l'acide nucléique-cible est un oligonucléotide dont la synthèse est obtenue chimiquement. La séquence de l'acide nucléique-cible est parfaitement aléatoire et peut être générée à l'aide d'un programme d'ordinateur, ou à l'aide de tout autre moyen.

Si l'on prend pour exemple un oligonucléotide de 50 nucléotides de long, la probabilité de retrouver une autre fois cette séquence dans la nature ou d'en faire la synthèse aléatoire au laboratoire une nouvelle fois est de 1 chance sur 1,26 10³⁰, soit un évènement hautement improbable. On peut rappeler ici, par exemple, que le génome humain haploïde entier a une taille de 3x10⁹ paires de base, environ.

Dans cette forme préférée de l'invention, la séquence complémentaire, c'est-à-dire celle de l'acide nucléique-sonde, est également synthétique et sert de sonde.

Si l'acide nucléique-sonde retrouve un acide nucléique-cible de séquence complémentaire, il se produit une hybridation avec formation d'un hybride ou duplex.

Cet hybride peut être détecté par tout moyen de détection approprié, connu dans le domaine des sondes nucléiques.

Ainsi, la détection de l'hybride ainsi formé peut s'effectuer après marquage ou non de la sonde.

Le marquage de la sonde peut être réalisé radioactivement ou non radioactivement par tout système de marquage approprié, connu dans le domaine des sondes nucléiques. Pour cela, on peut, par exemple, utiliser les procédés de marquage décrits dans les publications suivantes :
- Langer, P.R. Waldrop, A.A. et Ward, D.C. (1981) Proc. Natl. Acad. Sci. USA 78. 6633-6637.
- Leary, J.I., Brigati, D.J. et Ward, D.C. (1983) Proc. Natl. Acad.SCI. USA 80, 4045-4049.
- Tchen, P., Fuchs, R.P.P., Sage, E., et Leng, M. (1984) Proc. Natl. Acad. Sci. USA 81, 3466-3470.
- Kenz, M. (1983) EMBO J. 2, 817-822.
- Kenz, M. et Kurz, C. (1984) Nucleic Acids Res. 12, 3435-3444.
- Jablonski, E, Moomaw, E.W., Tullis, R.II. et Ruth, J.L. (1986) Nucleic Acids Res. 14, 6115-6128.
- Traincard, F., Ternyck, T., Dauchin, A. et Avrameas, S. (1983) Ann, Immunol. (Inst. Pasteur) 134 D. 399-405.

Dans le cas où la sonde n'est pas marquée, la détection de l'hybride peut être faite de manière spécifique ou non spécifique, à l'aide d'anticorps détectant les acides nucléiques simple ou double brin ou de protéines ou de composés présentant une affinité pour les acides nucléiques.

Par exemple, on peut faire appel, pour la détection, à des anticorps anti-hybrides. Il s'agit d'anticorps liés à une enzyme de détection qui sont incapables de se fixer sur un acide nucléique simple brin mais qui, en revanche, se fixent sur un acide nucléique double brin, comme c'est le cas d'un hybride.

Par conséquent, s'il y a formation d'un hybride, l'anticorps anti-hybride se fixe à cet hybride et il y a alors révélation par l'enzyme de détection.

Pour réaliser la détection de l'hybride, on peut faire appel, non seulement à des moyens chimiques, mais également à des moyens physiques, par exemple des moyens électriques, électroniques, radioélectriques ou magnétiques.

On peut notamment utiliser un système de détection piézo-électrique qui émet un signal particulier lorsqu'il y a hybridation. Ce signal peut être détecté et amplifié par tout système électronique approprié.

Dans une forme de réalisation préférée de l'invention, la sonde est marquée par un procédé de marquage non radioactif. Avantageusement, le marquage et la révélation sont non radioactifs et utilisent des marquages à la biotine, par sulfonation ou encore le procédé de marquage décrit dans la Demande de Brevet français n° 88 09598 et la Demande de Certification d'Addition n° 89 03192 déposées par la demanderesse.

L'acide nucléique-cible est, de préférence, répandu sur le support, ou directement sur l'objet si cela est possible, à l'aide d'un moyen applicateur approprié.

Comme moyen applicateur on peut utiliser un stylo (stylo à plume, stylo à bille, stylo feutre), un instrument à tracer pour le dessin, un pinceau, ou encore une machine automatisée comme une machine à jet d'encre.

Dans une forme de réalisation préférée de l'invention, cette application se fait à l'aide d'un stylo dans la cartouche duquel on a placé une solution de l'acide nucléique-sonde dans un tampon adéquat permettant une bonne fluidité et une bonne conservation de l'acide nucléique. La concentration est calculée de telle manière qu'il soit aisé de révéler par la suite l'acide nucléique-cible et qu'il soit facile d'écrire avec le stylo.

A titre d'exemple, cette concentration est telle que le moyen applicateur dépose de 10 pg à 10 ng d'acide nucléique-cible par mm². De façon préférentielle, une quantité de 1 ng/mm² semble avantageuse.

A titre d'exemple, on peut utiliser le tampon suivant :
Tris 50 mM pH : 8
EDTA 20 mM
Tween 20 0,3 %
NaCl 25 mM
Azide de sodium 0,04 % (ou autre conservateur).

Dans une forme préférée de l'invention, un numéro à 5 chiffres est écrit avec la solution d'acide nucléique et à l'aide du stylo, sur le support ou, le cas échéant, directement sur l'objet lui-même. Après séchage, le numéro est parfaitement invisible et ne pourra être révélé qu'à l'aide de l'acide nucléique-sonde de séquence complémentaire à celle de l'acide nucléique-cible qui est seule connue par le propriétaire de l'objet.

Pour assurer la révélation, les conditions d'hybridation sont importantes, en particulier avec les oligonucléotides. En effet, les cinétiques d'hybridation sont dépendantes de la concentration en ions sodium du milieu, de la nature du milieu et de la température d'hybridation. Cette température est elle-même dépendante de la stabilité des hybrides à former. Cette stabilité enfin est directement reliée à la teneur en base guanine et cytosine (GC) de l'acide nucléique.

A partir de la séquence nucléotidique aléatoire obtenue, destinée à constituer l'acide nucléique-cible, on peut calculer le pourcentage en base guanine et cytosine de l'oligonucléotide et définir ainsi les conditions optimales d'hybridation.

Ce sont ces conditions et elles seules qui seront utilisées pour la révélation.

Dans une autre variante de l'invention, on peut utiliser des moyens de détection non spécifiques de l'acide nucléique-cible. Cela signifie que de tels moyens sont capables de reconnaître, de manière non spécifique, une séquence d'acide nucléique-cible.

Bien évidemment, pour apporter la preuve irréfutable de la propriété d'un objet marqué, conformément au procédé de l'invention, il est tout particulièrement souhaitable de faire appel à des moyens de détection spécifiques, comme décrit précédemment.

Sous un autre aspect, l'invention concerne un dispositif de marquage crypté de haute sécurité pour la protection d'objets de valeur, comportant un composé chimique-cible propre à être détecté ultérieurement par un moyen de détection approprié.

Selon l'invention, ce dispositif est caractérisé en ce qu'il comprend :
- une solution d'un acide nucléique-cible de séquence choisie, formant motif de marquage et possédant un degré de fluidité choisie,
- un moyen applicateur propre à étendre ladite solution sur une surface à marquer, et
- un moyen de détection de l'acide nucléique.

Avantageusement, ladite solution contient en outre un tampon propre à conserver l'acide nucléique-cible et à conférer à cette solution le degré de fluidité suffisant pour permettre son emploi dans le moyen applicateur.

Ce moyen applicateur peut être, par exemple, un stylo, un pinceau, un instrument à dessin ou une machine automatisée, comme déjà indiqué précédemment.

Avantageusement, le dispositif de l'invention comprend en outre un support formant surface à marquer pour la solution et propre à être incorporé dans l'objet à marquer.

Ce support est formé d'une matière choisie parmi celles énumérées plus haut.

Le support peut être incorporé à une feuille d'un document, par exemple en fenêtre ou en bas de page, par estampage et/ou par collage au moyen d'une bande hologramme de sécurité.

On peut réaliser ainsi différents types de documents, en particulier des pages de contrat propres à recevoir sur un ou plusieurs supports disposés de façon appropriée les signatures des co-contractants. Si on essaye de falsifier le document en remplaçant un support par un autre, la bande hologramme se déforme et on voit immédiatement qu'il y a eu tentative de fraude. La bande hologramme est avantageusement une bande d'aluminium de faible épaisseur qui a subi un traitement d'holographie approprié.

Dans la description qui suit, faite seulement à titre d'exemple, on se réfère au dessin annexé, sur lequel :
- la figure 1 représente un support propre à être marqué selon le procédé de l'invention, au moyen d'un stylo applicateur ;
- la figure 2 représente le même support sous forme de rouleau ;
- la figure 3 représente le support ainsi enroulé avant introduction dans un logement approprié ménagé dans l'objet à protéger ;
- la figure 4 représente un document incorporant un support selon l'invention ;
- la figure 5 représente un document incorporant deux supports selon l'invention ;
- la figure 6 est une vue en coupe à échelle agrandie suivant la ligne VI-VI de la figure 5 ; et
- la figure 7 représente un document incorporant deux supports selon l'invention, dans une autre variante de réalisation.

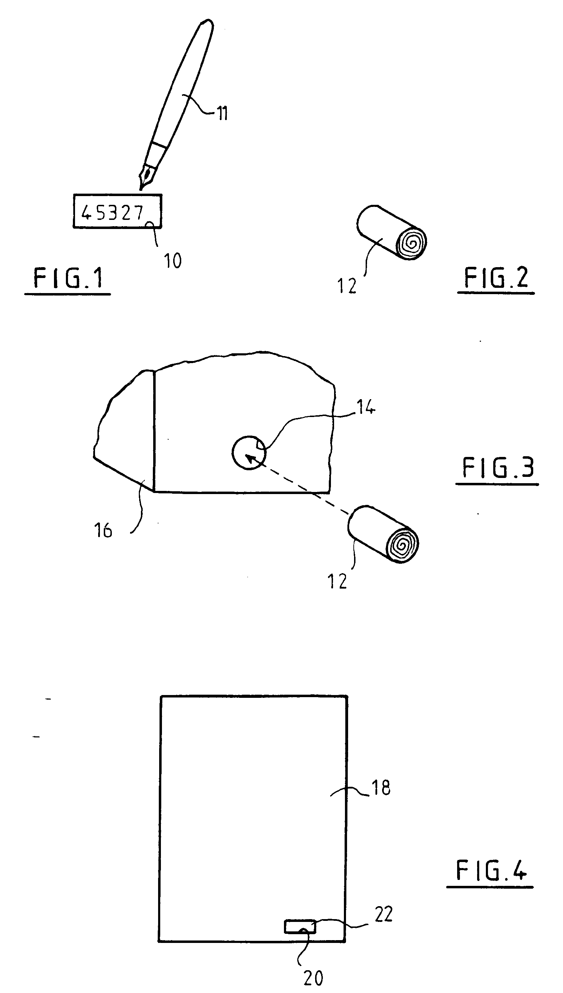

On se réfère tout d'abord à la figure 1 qui représente un support 10 selon l'invention constitué, dans l'exemple, par une membrane de forme rectangulaire de 5 sur 15 mm, représentant une surface d'environ 75 mm². Ce support est avantageusement constitué par une membrane de polyamide (nylon) ou encore de nitrocellulose.

On peut, par exemple, utiliser des membranes telles que celles commercialisées sous la marque ZETA-PROBE ou sous la marque ZETA-BIND par la société CUNO (Grande-Bretagne). On peut également utiliser des membranes nylon telles que celles commercialisées sous les marques GENE-SCREEN NORMAL ou GENE-SCREEN PLUS par la firme DUPONT-de-NEMOURS (Etats-Unis).

On peut aussi utiliser une membrane nylon telle que celle commercialisée sous la marque HY-BOND par la firme AMERSHAM (Grande-Bretagne).

On peut aussi utiliser une membrane nitrocellulose de la firme SCHLEICHER et SCHUELL (R F A ).

Sur la membrane 10 de la figure 1, on applique, au moyen d'un stylo 11, une solution d'acide nucléique-cible préparée comme indiqué précédemment. Dans l'exemple, on écrit sur cette membrane un numéro à cinq chiffres connu seulement du propriétaire de l'objet. Après séchage de la solution, le numéro ainsi appliqué sur le support est parfaitement invisible.

Ensuite, dans l'exemple considéré, on enroule la membrane 10 pour former un rouleau 12, tel que représenté sur la figure 2, qui occupe un volume extrêmement réduit puisque la surface du support à plat est d'environ 75 mm² et que l'épaisseur de la membrane est de quelques dizièmes de mm. Une fois la membrane ainsi roulée, le volume occupé par le rouleau 12 est de l'ordre de quelques mm³. Le rouleau 12 ainsi formé peut être caché dans l'objet à protéger et cela dans un endroit connu seulement du propriétaire.

Comme montré à la figure 3, on peut forer, à l'aide d'une mini-perçeuse, un trou 14 dans une région de l'objet 16 à protéger et, ensuite, insérer le rouleau 12 dans le trou ainsi formé. Il suffit ensuite de boucher le trou par un enduit ou mastic approprié pour ne laisser aucune trace visible de la perforation précédemment effectuée.

On se réfère maintenant à la figure 4 qui montre un document 18, par exemple une feuille de papier à lettre, de forme générale rectangulaire qui comporte, en partie inférieure, une fenêtre 20 de forme rectangulaire. Dans la fenêtre 20, est insérée une membrane 22 de nitrocellulose ou de nylon, chargée positivement ou non. On peut ainsi, sur la membrane 22, écrire, signer ou apposer toute sorte de signes de reconnaissance. Là encore, ces signes, lettres, mots, phrases, signatures sont parfaitement invisibles après séchage.

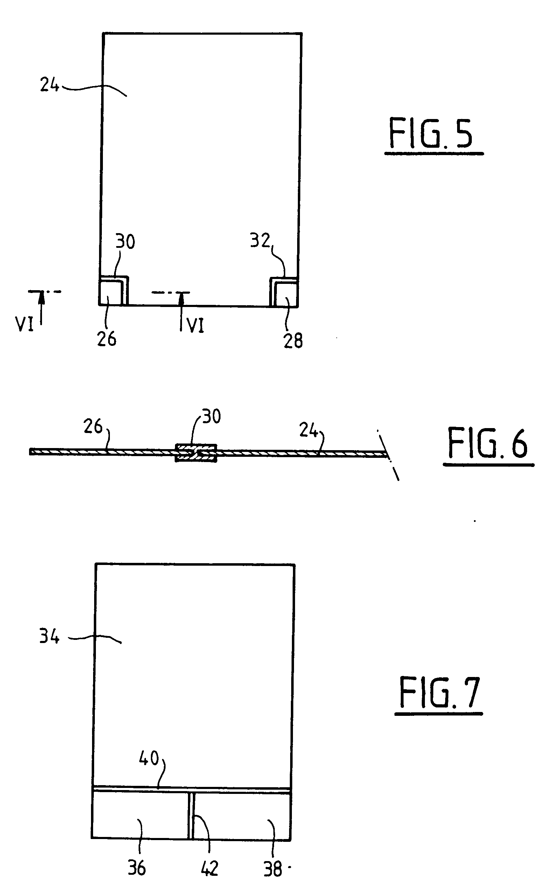

On se réfère maintenant à la figure 5 qui montre un document 24, par exemple une feuille de papier à lettre ou une feuille de contrat, comportant en bas de page deux membranes 26 et 28 en nitrocellulose ou en nylon formant support selon l'invention. Ces deux membranes sont des carrés de quelques millimètres de côté et sont disposées dans les coins inférieurs du document qui ont été découpés à cet effet. Chacune des membranes est fixée au document par une bande hologramme 30, 32 comme montré plus particulièrement à la figure 6. Chacune des bandes hologrammes est avantageusement une feuille en aluminium de faible épaisseur qui a été traitée par holographie et qui est estampée et/ou collée pour assurer la fixation de la membrane. Chaque bande comprend deux parties appliquées respectivement au recto et au verso du document.

Dans la forme de réalisation de la figure 7, le document 34 est muni en bas de page de deux membranes 36, 38 de forme rectangulaire qui, à elles deux, s'étendent sur toute la largeur du document. Les deux membranes sont reliées au document 34 par une bande hologramme 40 qui s'étend sur toute la largeur du document et elles sont reliées entre elles par une bande hologramme 42 qui peut avoir une longueur de quelques centimètres.

Les membranes 26, 28, 36 et 38 peuvent servir à recevoir les signatures de co-contractants.

L'acide nucléique-cible ainsi appliqué sur la membrane 10 de la figure 1, sur la membrane 22 de la figure 4, sur les membranes 26 et 28 de la figure 5 ou sur les membranes 36 et 38 de la figure 7 ne peut être révélé qu'à l'aide de la sonde complémentaire adéquate pour identifier ou authentifier soit l'objet 16 de la figure 3, soit les documents 18, 24 et 34 des figures 4, 5 et 7. Ce document peut être un acte, un contrat, du papier monnaie, un chèque, une action, un testament, etc.

L'invention procure donc une triple sécurité au propriétaire de l'objet à marquer par le procédé selon l'invention.

Tout d'abord, le support (membrane nylon par exemple) peut être caché dans un endroit connu seulement du propriétaire. La cache n'est pas détectable par les rayons X puisque la membrane et l'acide nucléique sont, l'un comme l'autre, parfaitement transparents aux rayons X ou aux rayons gamma. Par ailleurs, le volume occupé par la membrane est extrêmement réduit puisque, à l'état roulé, celui-ci est de l'ordre de quelques mm³.

En second lieu, seul le propriétaire de l'objet connaît le numéro écrit sur le support.

En troisième lieu, la révélation de l'hybride ne peut se faire qu'avec la sonde nucléique de séquence complémentaire, connue seulement par le propriétaire, et cela dans des conditions d'hybridation définies par la concentration en bases guanine et cytosine de l'hybride. Or, cette teneur est déterminée à partir de la séquence nucléique-cible connue seulement du propriétaire de l'objet à protéger.

La révélation peut être faite en présence d'un huissier ou d'un représentant assermenté garantissant le déroulement normal de l'opération de détection et l'utilisation de la sonde complémentaire spécifique détenue par le propriétaire de l'objet à protéger.

Dans le cas où l'objet à protéger est un papier ou un document, il est aisé d'y inclure des fragments de support, par exemple de membranes de nylon, et d'y écrire tout code numéroté, signature ou signe de reconnaissance permettant d'identifier le document.

Le papier servant à la confection du document peut être un papier ordinaire ou un papier spécial, par exemple un papier fluorescent de sécurité.

On connaît en effet de tels papiers qui comportent des motifs fluorescents si bien que lorsqu'on les irradie par une lampe à ultraviolet, ils émettent des rayonnements de différentes couleurs.

## Revendications

1. Procédé de marquage crypté de haute sécurité pour la protection d'objets de valeur, dans lequel on applique sur un objet à marquer un composé chimique-cible propre à être détecté ultérieurement par un moyen de détection approprié, caractérisé en ce qu'il comprend les opérations consistant à :
a) préparer une solution d'un acide nucléique-cible de séquence choisie formant motif de marquage et possédant un degré de fluidité choisi,
b) incorporer une quantité choisie de ladite solution à chaque objet d'une classe d'objets à marquer,
ce qui permet de procéder ultérieurement, en cas de besoin, à une opération
c) d'identification ou d'authentification de l'objet en utilisant un moyen de détection dudit acide nucléique pour révéler le marquage.

2. Procédé selon la revendication 1, caractérisé en ce que l'opération b) consiste à appliquer la solution d'acide nucléique sur une surface d'un support pour l'imprégner de ladite solution et à incorporer ensuite le support ainsi imprégné dans chaque objet à marquer.

3. Procédé selon la revendication 2, caractérisé en ce que le support est en nitrocellulose, en nylon chargé positivement ou non chargé, en papier, en carton, en bois, en matière plastique, en tissu, en métal, en verre, en céramique, en terre cuite, en une matière organique sous forme de billes ou de gel, ou encore en une matière minérale.

4. Procédé selon l'une des revendications 2 et 3, caractérisé en ce que le support est une membrane de petite dimension susceptible d'être insérée à plat dans un objet mince ou susceptible d'être enroulée pour former un rouleau propre à être inséré dans un objet épais.

5. Procédé selon la revendication 1, caractérisé en ce que l'opération b) consiste à appliquer la solution d'acide nucléique directement sur une surface de l'objet à marquer, dans le cas où le matériau constitutif de ladite surface permet un accrochage direct dudit acide nucléique.

6. Procédé selon la revendication 1, caractérisé en ce que, dans l'opération b), on applique la solution d'acide nucléique sous la forme d'un marquage en écrivant un numéro, une lettre, un mot, une phrase, une signature ou tout autre type de marquage.

7. Procédé selon l'une des revendications 1 à 6, caractérisé en ce que, dans l'opération c), on utilise, comme moyen de détection, une sonde d'acide nucléique simple brin de séquence complémentaire de celle de l'acide nucléique-cible incorporée à l'objet et capable de s'hybrider à celle-ci, ce qui permet une détection spécifique de l'acide nucléique-cible par hybridation moléculaire.

8. Procédé selon la revendication 7, caractérisé en ce que l'on détecte ensuite l'hybride à l'aide de composés radioactifs, de composés non radioactifs, de moyens à détection directe, de moyens à détection indirecte, de moyens chimiques ou encore de moyens physiques, par exemple de moyens électriques, électroniques, radioélectriques ou magnétiques.

9. Procédé selon l'une des revendications 1 à 8, caractérisé en ce que l'acide nucléique-cible et l'acide nucléique-sonde sont choisis parmi de l'ADN ou de l'ARN, d'origine naturelle ou synthétique, parmi des dérivés de synthèse contenant en tout ou en partie des bases rares ou des bases modifiées, parmi des séquences nucléiques ou des génomes susceptibles de générer un polymorphisme, ou encore parmi des séquences de génome ou des génomes entiers de personnes humaines.

10. Procédé selon la revendication 9, caractérisé en ce que l'acide nucléique-cible et l'acide nucléique-sonde sont des oligonucléotides complémentaires dont les séquences sont obtenues de façon aléatoire, par exemple au moyen d'un programme informatique.

11. Procédé selon l'une des revendications 7 à 10, caractérisé en ce qu'on réalise l'hybridation dans des conditions spécifiques dépendant de la teneur en bases guanine et cytosine de la séquence de l'acide nucléique-cible, générée aléatoirement.

12. Procédé selon l'une des revendications 1 à 11, caractérisé en ce que la solution d'acide nucléique-cible contient un tampon propre à conserver l'acide nucléique et à assurer à la solution le degré de fluidité suffisant pour permettre son application et en ce qu'on utilise un moyen applicateur pour étendre ladite solution et former des symboles sur une surface à marquer.

13. Procédé selon l'une des revendications 1 à 12, caractérisé en ce que, pour l'opération c), on utilise des moyens de détection non spécifiques.

14. Dispositif de marquage crypté de haute sécurité pour la protection d'objets de valeur, caractérisé en ce qu'il comprend :
- une solution d'un acide nucléique-cible de séquence choisie, formant motif de marquage et possédant un degré de fluidité choisi,
- un moyen applicateur propre à étendre ladite solution sur une surface à marquer,
- un moyen de détection de l'acide nucléique.

15. Dispositif selon la revendication 14, caractérisé en ce que ladite solution contient en outre un tampon propre à conserver l'acide nucléique-cible et à conférer à cette solution le degré de fluidité suffisant pour permettre son emploi dans le moyen applicateur.

16. Dispositif selon la revendication 15, caractérisé en ce que la concentration de l'acide nucléique-cible dans la solution est telle que le moyen applicateur dépose de 10 pg à 10 ng d'acide nucléique par mm² sur la surface à marquer.

17. Dispositif selon l'une des revendications 14 à 16, caractérisé en ce que le moyen applicateur est un stylo à bille, un stylo à plume, un stylo feutre, un pinceau, un instrument de dessin, ou une machine automatisée, par exemple une machine à jet d'encre.

18. Dispositif selon l'une des revendications 14 à 17, caractérisé en ce qu'il comprend, en outre, un support formant surface à marquer pour la solution et propre à être incorporé dans l'objet à marquer.

19. Dispositif selon la revendication 18, caractérisé en ce que le support est en nitrocellulose, en nylon chargé positivement ou non chargé, en papier, en carton, en bois, en matière plastique, en tissu, en métal, en verre, en céramique, en terre cuite, en une matière organique sous forme de bille ou de gel, ou encore en une matière minérale.

20. Dispositif selon l'une des revendications 18 et 19, caractérisé en ce que le support est incorporé à une feuille d'un document, par exemple en fenêtre ou en bas de page, par estampage et/ou collage au moyen d'une bande hologramme.

21. Dispositif selon la revendication 14, caractérisé en ce que le moyen de détection est spécifique de l'acide nucléique-cible.

22. Dispositif selon la revendication 14, caractérisé en ce que le moyen de détection est non spécifique de l'acide nucléique-cible.

## Claims

1. Process for high security coded marking for the protection of valuable objects, in which there is applied to an object to be marked a target chemical compound capable of being subsequently detected by a suitable detection means, characterized in that it comprises the operations consisting in :
a) preparing a solution of a target nucleic acid of chosen sequence forming a marking unit and possessing a chosen degree of fluidity,
b) incorporating a chosen quantity of the said solution in each object of a class of objects to be marked,
which makes it possible to perform subsequently, if need be, an operation
c) of identification or of authentication of the object by using a means of detection of the said nucleic acid to reveal the marking.

2. Process according to Claim 1, characterized in that the operation b) consists in applying the nucleic acid solution to a surface of a support in order to impregnate it with the said solution, and in then incorporating the support thus impregnated into each object to be marked.

3. Process according to Claim 2, characterized in that the support is made of nitrocellulose, positively charged or uncharged nylon, paper, cardboard, wood, plastic, cloth, metal, glass, ceramic, terracotta, an organic material in the form of beads or gel or else an inorganic material.

4. Process according to one of Claims 2 and 3, characterized in that the support is a membrane of small size which can be inserted flat into a thin object or which can be rolled up to form a scroll capable of being inserted into a thick object.

5. Process according to Claim 1, characterized in that the operation b) consists in applying the nucleic acid solution directly to a surface of the object to be marked, in the case where the constituent material of the said surface allows a direct attachment of the said nucleic acid.

6. Process according to Claim 1, characterized in that, in the operation b), the nucleic acid solution is applied in the form of a marking by writing a number, a letter, a word, a phrase, a signature or any other type of marking.

7. Process according to one of Claims 1 to 6, characterized in that, in the operation c), a single-stranded nucleic acid probe, of sequence complementary to that of the target nucleic acid incorporated in the object and capable of hybridizing with the latter sequence, is used as a detection means, which allows specific detection of the target nucleic acid by molecular hybridization.

8. Process according to Claim 7, characterized in that the hybrid is then detected with the aid of radioactive compounds, non-radioactive compounds, means of direct detection, means of indirect detection, chemical means or else physical means, for example electrical, electronic, radioelectric or magnetic means.

9. Process according to one of Claims 1 to 8, characterized in that the target nucleic acid and the probe nucleic acid are chosen from DNA or RNA, of natural or synthetic origin, from synthetic derivatives wholly or partially containing rare bases or modified bases, from nucleic acid sequences or from genomes which can generate a polymorphism, or else from genome sequences or entire genomes of human beings.

10. Process according to Claim 9, characterized in that the target nucleic acid and the probe nucleic acid are complementary oligonucleotides whose sequences are obtained randomly, for example by means of a computer program.

11. Process according to one of Claims 7 to 10, characterized in that the hybridization is carried out under specific conditions which depend on the content of guanine and cytosine bases in the target nucleic acid sequence, which is randomly generated.

12. Process according to one of Claims 1 to 11, characterized in that the target nucleic acid solution contains a buffer capable of preserving the nucleic acid and of providing the solution with a sufficient degree of fluidity to allow its application, and in that an applicator means is used to spread the said solution and to form symbols on a surface to be marked.

13. Process according to one of Claims 1 to 12, characterized in that, for the operation c), non-specific detection means are used.

14. High security coded marking device for the protection of valuable objects, characterized in that it comprises :
- a target nucleic acid solution of chosen sequence, forming a marking unit and possessing a chosen degree of fluidity,
- an applicator means capable of spreading the said solution on a surface to be marked,
- a means for detection of the nucleic acid.

15. Device according to Claim 14, characterized in that the said solution contains in addition a buffer capable of preserving the target nucleic acid and of conferring on this solution a sufficient degree of fluidity to allow its use in the applicator means.

16. Device according to Claim 15, characterized in that the concentration of the target nucleic acid in the solution is such that the applicator means deposits from 10 pg to 10 ng of nucleic acid per mm² on the surface to be marked.

17. Device according to one of Claims 14 to 16, characterized in that the applicator means is a ball-point pen, a fountain pen, a felt-tip pen, a paintbrush, a drawing instrument or an automated machine, for example an ink-jet machine.

18. Device according to one of Claims 14 to 17, characterized in that it comprises, in addition, a support forming a surface to be marked for the solution and capable of being incorporated into the object to be marked.

19. Device according to Claim 18, characterized in that the support is made of nitrocellulose, positively charged or uncharged nylon, paper, cardboard, wood, plastic, cloth, metal, glass, ceramic, terracotta, an organic material in the form of beads or gel or else an inorganic material.

20. Device according to one of Claims 18 and 19, characterized in that the support is incorporated in a sheet of a document, for example in a window or at the foot of a page, by stamping and/or sticking by means of a hologram strip.

21. Device according to Claim 14, characterized in that the detection means is specific for the target nucleic acid.

22. Device according to Claim 14, characterized in that the detection means is non-specific for the target nucleic acid.

## Patentansprüche

1. Verfahren zur verborgenen Sicherheitsmarkierung zum Schutz von Wertgegenständen, in welchem auf einen zu markierenden Gegenstand eine chemische Zielverbindung aufgetragen wird, die sich dazu eignet, später durch ein geeignetes Nachweismittel nachgewiesen zu werden, **dadurch gekennzeichnet,** daß es die Schritte umfaßt:
a) Herstellen einer Lösung einer Zielnukleinsäure mit ausgewählter Sequenz, welche das Markierungs-Motiv bildet und einen ausgewählten Fluiditätsgrad besitzt,
b) Inkorporieren einer gewählten Menge der Lösung in jeden Gegenstand einer Klasse von zu markierenden Gegenständen, was es ermöglicht, später, falls dies erforderlich ist, einen Schritt
c) der Identifizierung oder des Nachweises der Echtheit des Gegenstandes unter Verwendung eines Nachweismittels der Nukleinsäure zum Aufdecken der Markierung durchzuführen.

2. Verfahren nach Anspruch 1, **dadurch gekennzeichnet,** daß der Schritt b) darin besteht, die Lösung der Nukleinsäure auf eine Oberfläche eines Trägers aufzutragen, um ihn mit der Lösung zu imprägnieren, und anschließend den so imprägnierten Träger in jeden zu markierenden Gegenstand zu inkorporieren.

3. Verfahren nach Anspruch 2, **dadurch gekennzeichnet,** daß der Träger aus Nitrocellulose, aus positiv geladenem oder ungeladenem Nylon, aus Papier, aus Pappe, aus Holz, aus Kunststoffmaterial, aus Gewebe, aus Metall, aus Glas, aus Keramik, aus Terrakotta, aus einem organischen Material in Form von Kugeln oder Gel, oder auch aus einem mineralischen Material ist.

4. Verfahren nach einem der Ansprüche 2 und 3, **dadurch gekennzeichnet**, daß der Träger eine Membran mit geringer Größe ist, welche flach in einen dünnen Gegenstand eingeführt werden kann, oder welche aufgerollt werden kann, um eine Rolle zu bilden, die sich dazu eignet, in einen dicken Gegenstand eingeführt zu werden.

5. Verfahren nach Anspruch 1, **dadurch gekennzeichnet**, daß der Schritt b) darin besteht, die Lösung der Nukleinsäure direkt auf eine Oberfläche des zu markierenden Gegenstandes aufzutragen, falls das Material, aus dem die Oberfläche gebildet ist, ein direktes Anbringen der Nukleinsäure erlaubt.

6. Verfahren nach Anspruch 1, **dadurch gekennzeichnet**, daß in Schritt b) die Nukleinsäurelösung in Form einer Markierung durch Schreiben einer Zahl, eines Buchstaben, eines Wortes, eines Satzes, einer Unterschrift oder jeder anderen Art von Markierung aufgetragen wird.

7. Verfahren nach einem der Ansprüche 1 bis 6, **dadurch gekennzeichnet**, daß im Schritt c) als Nachweismittel eine Sonde aus einzelsträngiger Nukleinsäure mit einer Sequenz, die zu der Sequenz der Zielnukleinsäure, die in den Gegenstand inkorporiert ist, komplementär ist, und die mit dieser hybridisieren kann, verwendet wird, was einen spezifischen Nachweis der Zielnukleinsäure durch molekulare Hybridisierung ermöglicht.

8. Verfahren nach Anspruch 7, **dadurch gekennzeichnet,** daß anschließend das Hybrid mittels radioaktiver Verbindungen, nichtradioaktiver Verbindungen, direkter Nachweismittel, indirekter Nachweismittel, chemischer Mittel oder auch physikalischer Mittel, z.B. elektrischer, elektronischer, radioelektrischer oder magnetischer Mittel nachgewiesen wird.

9. Verfahren nach einem der Ansprüche 1 bis 8, **dadurch** **gekennzeichnet,** daß die Zielnukleinsäure und die Nukleinsäuresonde ausgewählt sind aus DNA oder RNA, natürlichen oder synthetischen Ursprungs, aus synthetisch gewonnenen Derivaten, die ganz oder teilweise seltene Basen oder modifizierte Basen enthalten, aus Nukleinsäuresequenzen oder Genomen, die einen Polymorphismus erzeugen können, oder auch aus Sequenzen des Genoms oder ganzen Genomen von menschlichen Personen.

10. Verfahren nach Anspruch 9, **dadurch gekennzeichnet,** daß die Zielnukleinsäure und die Nukleinsäuresonde komplementäre Oligonukleotide sind, deren Sequenzen in zufälliger Weise, z.B. mittels eines Computerprogramms, erhalten werden.

11. Verfahren nach einem der Ansprüche 7 bis 10, **dadurch** **gekennzeichnet,** daß die Hybridisierung unter spezifischen Bedingungen durchgeführt wird, die vom Gehalt an den Basen Guanin und Cytosin der Zielnukleinsäuresequenz, welche in zufälliger Weise erzeugt wurde, abhängen.

12. Verfahren nach einem der Ansprüche 1 bis 11, **dadurch** **gekennzeichnet,** daß die Zielnukleinsäurelösung einen Puffer enthält, der sich dazu eignet, die Nukleinsäure zu konservieren und einen Fluiditätsgrad der Lösung zu gewährleisten, der ausreichend ist, um ihre Auftragung zu ermöglichen, und dadurch, daß ein Auftragungsmittel zum Ausbreiten der Lösung und zum Bilden von Symbolen auf einer zu markierenden Oberfläche verwendet wird.

13. Verfahren nach einem der Ansprüche 1 bis 12, **dadurch** **gekennzeichnet,** daß für Schritt c) nichtspezifische Nachweismittel verwendet werden.

14. Vorrichtung zur verdeckten Sicherheitsmarkierung zum Schutz von Wertgegenständen, **dadurch gekennzeichnet,** daß sie umfaßt:
eine Lösung einer Zielnukleinsäure mit ausgewählter Sequenz, welche das Markierungs-Motiv bildet und einen ausgewählten Fluiditätsgrad besitzt,
ein Auftragungsmittel, welches sich dazu eignet, die Lösung auf einer zu markierenden Oberfläche auszubreiten,
ein Nachweismittel der Nukleinsäure.

15. Vorrichtung nach Anspruch 14, **dadurch gekennzeichnet,** daß die Lösung außerdem einen Puffer enthält, der sich dazu eignet, die Zielnukleinsäure zu konservieren und der Lösung den Fluiditätsgrad zu verleihen, der ausreichend ist, um ihren Einsatz in dem Auftragungsmittel zu ermöglichen.

16. Vorrichtung nach Anspruch 15, **dadurch gekennzeichnet,** daß die Konzentration der Zielnukleinsäure in der Lösung so ist, daß das Auftragungsmittel 10 pg bis 10 ng Nukleinsäure pro mm² auf der zu markierenden Oberfläche abscheidet.

17. Vorrichtung nach einem der Ansprüche 14 bis 16, **da****durch gekennzeichnet,** daß das Auftragungsmittel ein Kugelschreiber, ein Federhalter, ein Filzstift, ein Pinsel, ein Zeichengerät oder eine automatisierte Maschine, z.B. ein Tintenstrahldrucker ist.

18. Vorrichtung nach einem der Ansprüche 14 bis 17, **da****durch gekennzeichnet,** daß sie außerdem einen Träger umfaßt, der eine zu markierende Oberfläche für die Lösung bildet und sich dazu eignet, in den zu markierenden Gegenstand inkorporiert zu werden.

19. Vorrichtung nach Anspruch 18, **dadurch gekennzeichnet,** daß der Träger aus Nitrocellulose, aus positiv geladenem oder ungeladenem Nylon, aus Papier, aus Pappe, aus Holz, aus Kunststoffmaterial, aus Gewebe, aus Metall, aus Glas, aus Keramik, aus Terrakotta, aus einem organischen Material in Form von Kugeln oder Gel, oder auch aus einem mineralischen Material ist.

20. Vorrichtung nach einem der Ansprüche 18 und 19, **da****durch gekennzeichnet,** daß der Träger in einen Bogen eines Dokumentes, z.B. in ein Fenster oder am Fuß der Seite, durch Prägedruck und/oder Aufkleben mittels eines Hologrammstreifens inkorporiert wird.

21. Vorrichtung nach Anspruch 14, **dadurch gekennzeichnet,** daß das Nachweismittel für die Zielnukleinsäure spezifisch ist.

22. Vorrichtung nach Anspruch 14, **dadurch gekennzeichnet,** daß das Nachweismittel für die Zielnukleinsäure nicht spezifisch ist.
